(19) Europäisches Patentamt
European Patent Office
Office européen des brevets

(11) Publication number: 0 520 130 B1

(12) **EUROPEAN PATENT SPECIFICATION**

(45) Date of publication of patent specification: **12.04.95** (51) Int. Cl.[6]: **C07C 273/18**

(21) Application number: **92100774.6**

(22) Date of filing: **17.01.92**

# (54) Process for preparing symmetric N, N'-disubstituted aromatic urea.

(30) Priority: **24.06.91 KR 1051391**

(43) Date of publication of application:
**30.12.92 Bulletin 92/53**

(45) Publication of the grant of the patent:
**12.04.95 Bulletin 95/15**

(84) Designated Contracting States:
**AT BE CH DE DK ES FR GB GR IT LI LU MC NL PT SE**

(56) References cited:
**EP-A- 0 217 166**

**CHEMICAL ABSTRACTS, vol. 115, no. 15, 14 October 1991, Columbus, Ohio, US; abstract no. 158745Z, page 896 ;**

**JOURNAL OF ORGANIC CHEMISTRY. vol. 40, no. 19, 1975, EASTON US pages 2819 - 2822; H. A. DIECK ET AL: 'A Low-pressure, Palladium-Catalyzed N,N'-Diarylurea Synthesis from Nitro Compounds, Amines, and Carbon Monoxide'**

(73) Proprietor: **LUCKY LTD.**
**20, Yoido-dong,**
**Yongdungpo-ku**
**Seoul 150-721 (KR)**

(72) Inventor: **Oh, Jae Seung**
**381-42, Doryong-Dong,**
**Lucky Apt. 6-402**
**Yoosung-Ku,**
**Daejeon (KR)**
Inventor: **Lee, Sang Moo**
**386-1, Doryong-Dong,**
**Lucky Apt. 312**
**Yoosung-Ku,**
**Daejeon (KR)**

(74) Representative: **Turi, Michael, Dipl.-Phys. et al**
**Samson & Partner**
**Widenmayerstrasse 5**
**D-80538 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 520 130 B1

## Description

BACKGROUND OF THE INVENTION

1. Field of the Invention

The present invention relates to a process for preparing N,N' -disubstituted urea; and, more particularly, to an improved process for preparing N,N' -disubstituted urea derivatives of the following formula(I)

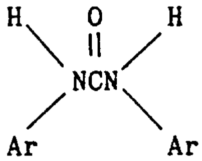

(I)

wherein Ar represents an aromatic radical optionally substituted with a halogen, alkyl, or alkoxy group, which comprises reacting an aromatic mononitro compound, an aromatic primary amine and synthesis gas in the presence of a catalyst consisting essentially of a divalent palladium compound as a main catalyst component and an ammonium or phosphonium salt containing a halogen atom as a co-catalyst component with or without a solvent.

2. Description of the Prior Art

The N,N' -disubstituted urea is an important intermediate for the production of carbamates which may be employed as raw materials for certain agrochemicals. Conventional methods for preparing N,N' -disubstituted urea have heretofore been developed through the reaction of amines with carbon monoxide in the presence of a non-metal catalyst such as tertiary aliphatic amine(J. Org. Chem., 26, 3309(1961)), selenium(J. Am. Chem. Sco., 93, 6344(1971)) or a metal catalyst such as cobalt carbonyl(Can. J. Chem., 40, 1718 (1962)), silver acetate(J. Org. Chem., 37, 2670(1972)) and the like.

The disclosure in J. Org. Chem., 26, 3309(1961) relates to a method to synthesize ureas using a tertiary aliphatic amine as a catalyst, and an excess amount of sulfur, which in turn produces an unwanted by-product, hydrogen sulfide; and its removal process entails an additional cost.

The selenium catalyst employed in the method disclosed in J. Am. Chem. Soc., 93, 6344(1971) has a well-known toxicity problem. A continuous supply of oxygen is also required in this method to precipitate the selenium catalyst, which is not only dangerous due to the possibilities of explosion, but also expensive.

U. S. Patent No. 4,052,454 also discloses a process for the production of ureas by synthesizing a disubstituted urea with a selenium or sulfur catalyst. The maxium yield of the process using the selenium catalyst is limited to 67.3%. In the case of using the sulfur catalyst, the urea yield given in the example is merely 3.4-7.7%; and the process is clearly impractical.

Other methods for preparing N, N' -disubstituted urea derivatives in the presence of metal catalysts except platinum group catalysts are not practical since the yield and selectivity of the reaction is quite low.

Processes using a platinum group catalyst are disclosed in European Patent Publication No. 0,319,111, Japanese Patent Publication No. 53-41,123, Japanese Patent Laid-Open Publication Nos. 58-144,363 and 62-59,253, and J. Org. Chem., 40, 2819(1975).

Among such disclosures, European Patent Publication No. 0,319,111 uses a salt of Cu, Fe, Mn, V, Cr, Zn, Sn, U, or Ce in addition to a palladium compound. It is well known that these salts promote the so-called Wacker-type reaction, which involves a redox cycle between Pd(II) and Pd(O) with a metal salt serving as a reoxidant of Pd(O) to active Pd(II) species. Hence, the presence of a reoxidant is essential in this type of reaction. Otherwise, the catalyst is separated as a zero-valent metal, and the efficiency of the reaction decreases. When an excess amount of amine is used, unsymmetric urea is mainly obtained in this method. A best example given in the specification gives a 73% yield of 1,1-dimethyl-3(4-chlorobenzene)-urea after 20 hours of reaction time.

Japanese Patent Publication No. 53-41,123 and Japanese Patent Laid-Open Publication No. 58-144,363 relate to the process for preparing N,N' -disubstituted urea by reacting amines with carbon monoxide and oxygen at an elevated temperature and a high pressure. In such methods, it is not only difficult to control the partial pressure of two kinds of gases involved, i.e., carbon monoxide and oxygen, but also there is a

danger of explosion due to the presence of oxygen.

The disclosure made in J. Org. Chem., 40, 2819(1975) provides a method to synthesize N,N' -diaryl urea at 1 atm, 90 ° C and with the initial amine/nitro compound molar ratio of less than 1. In the disclosure, tri-n-butyl amine is used up to 50% together with a solvent to maintain the catalytic activity. Otherwise, the activity of the palladium catalyst is suddenly decreased during the reaction. Since a small amount of aromatic primary amine is employed, the decomposition of catalyst is not effectively inhibited. Hence, the catalyst cannot be reused and recycled, and the activity of the catalyst is reduced to a 50% level of the initial activity after one cycle of the reaction. Although it is ideal for this reaction to dissolve the palladium catalyst completely in the aromatic primary amine, it is actually very difficult to achieve it since the amine concentration is low in the reaction mixture. Furthermore, the reaction pressure is too low to conduct an efficient carbonylation reaction. As a result, the yield of the aromatic urea according to the method is as low as 64%.

The process disclosed in Japanese Patent Laid-Open Publication No. 62-59,253 gives a relatively high yield and selectivity. However, it requires expensive catalysts such as rhodium and ruthenium compounds. Furthermore, the appearance of the resulting N,N' -disubstituted urea is not neat, and the catalysts are unstable at a high temperature and decomposed around the reaction temperature.

All the methods discussed above employ pure carbon monoxide as the raw material. Since carbon monoxide is usually separated from synthesis gas by cryogenic distillation or solvent absorption, additional investments for the purification of carbon monoxide are required. On the other hand, the synthesis gas itself, consisting of mainly carbon monoxide and hydrogen in various proportions, can be manufactured cheaply by such methods as coal gasification, partial oxidation or steam cracking of natural gas and oil, etc. If the synthesis gas is used instead of pure carbon monoxide as the raw material for urea synthesis, it will be superior in terms of economy to any other processes developed so far. Furthermore, if the hydrogen in synthesis gas reacts with a mononitro compound producing a corresponding amine during the synthesis of a urea compound such as formula(I), it can reduce the consumption of the amine which is more expensive than the corresponding mononitro compound; and, consequently, the process economy will be greatly improved.

In this connection, Inorg. Chem., 9, 342(1970) does disclose a method to synthesize N,N' -disubstituted urea by reacting nitrobenzene with synthesis gas. However, this method requires a high pressure of carbon monoxide; and the yield of urea is very low.

## SUMMARY OF THE INVENTION

Accordingly, the primary object of the present invention is to provide an improved process for preparing N,N' -disubstituted urea derivatives in a high yield.

More particularly, the present invention provides a process for the preparation of N',N' -disubstituted urea which comprises reacting an aromatic mononitro compound and an aromatic primary amine with synthesis gas in the presence of a catalyst consisting essentially of a palladium compound as a main catalyst and an ammonium or phosphonium salt as a cocatalyst wherein the molar ratio of the aromatic primary amine to the mononitro compound in the reaction mixture is greater than two so as to: attain a high yield of the desired product; inhibit catalyst decompositon during the reaction; and recover the catalyst maintaining the initial activity without any further treatment.

The present invention further provides a process for the preparation of N,N' -disubstituted urea derivatives which comprises reacting an aromatic primary amine, an aromatic mononitro compound, and synthesis gas in the presence of a catalyst wherein the molar ration of hydrogen to carbon monoxide in the synthesis gas is less than 5, the aromatic primary amine is used both as a reactant and as a solvent so as to wash the product solids for the complete recovery of the catalyst, the reaction temperature lies within the range from 50 to 200 ° C, and the reaction pressure ranges from 5 to 100 atm.

## BRIEF DESCRIPTION OF THE DRAWING

The present invention will become more fully understood from the detailed description given hereinbelow with reference to the accompanying drawing, which is given by way of illustration only and thus not limitative of the present invention.

Fig. 1 shows a schematic diagram of the continuous process of the present invention for preparing N,N' -disubstituted urea, although the process can be carried out in either a batchwise or a continuous mode.

DETAILED DESCRIPTION OF THE INVENTION

Referring now to Fig. 1, the process for preparing N,N' - disubstituted urea of the formula(I), as shown in the drawing, comprises reacting an aromatic mononitro compound, an aromatic primary amine, and synthesis gas in the presence of a catalyst consisting essentially of a palladium compound as a main catalyst and an ammonium or phosphonium salt containing a halogen atom as a co-catalyst with or without a solvent.

The aromatic mononitro compound is selected from the group consisting of nitrobenzenes, nitronaphthalenes, nitroanthracenes and nitrobiphenyls. Exemplary compounds thereof include: nitrobenzene, o-, m-, or p-nitrotoluene, o-nitro-p-xylene, 2-methyl-1-nitronaphthalene,o-, m- or p-chloronitrobenzene, 1-bromo-4-nitrobenzene, 2-chloro-6-nitrotoluene, 4-chloro-3-nitrotoluene, 1,4-dichloro-2-nitrobenzene, 3,4-dichloro-1-nitrobenzene, alpha-chloro-m-nitrotoluene, 1,2,4,-trichloro-5-nitrobenzene, etc.

The aromatic primary amine is selected from the group consisting of anilines, aminonaphthalenes, aminoanthracenes and aminobiphenyls which correspond to the aromatic mononitro compounds. Their representative compounds include aniline, o-, m- or p-toluidine, o-, m-, p-chloroaniline, alpha- or beta-naphthylamine, 2-methyl-1-aminonaphthalene, aminotoluene, etc.

Since the primary amine used in the present invention functions not only as a reactant but also as a solvent, it is preferred to use it greater than twice the moles of the mononitro compound so that it inhibits the decomposition of the catalyst and makes it easier to recover the catalyst thanks to its ability to wash the product solids.

Two reaction schemes(1) and (2) shown below compete for the production of N,N' -disubstituted urea of the formula(I):

$$Ar^1NH_2 + Ar^2NO_2 + 3CO \longrightarrow Ar^1NH\overset{O}{\overset{\|}{C}}NHAr^2 + 2CO_2 \qquad (1)$$

$$5Ar^1NH_2 + Ar^2NO_2 + 3CO \longrightarrow Ar^1NH\overset{O}{\overset{\|}{C}}NHAr2 + 2Ar^1NH\overset{O}{\overset{\|}{C}}NHAr^1 + 2H_2O \qquad (2)$$

wherein Ar is the same as defined above; and $Ar^1$ and $Ar^2$ are employed to denote their sources: that is, $Ar^1$ represents the aromatic primary amine; and $Ar^2$ indicates the aromatic mononitro compound.

The production rate of N,N' -disubstituted urea depends on the molar ratio of the aromatic mononitro compound to the aromatic primary amine. As shown in the above equations(1) and (2), when the amount of mononitro compound is constant, the larger the amount of aromatic primary amine is, the larger the amount of N,N' -disubstituted urea is produced. It is because the reaction rate of the equation(2) is increased more rapidly than the reaction rate of the equation(1) when the concentration of the aromatic primary amine increases.

The synthesis gas used in the present invention does not have any particular limitation in terms of its soure or manufacturing process. It is sufficient to purify the synthesis gas by a known procedure to remove such components as sulfur compound, carbon dioxide, ammonia, etc., which may contaminate the catalyst. Especially, there is no need to remove nitrogen in the synthesis gas since nitrogen is not involved in the reaction.

The molar ratio of hydrogen to carbon monoxide in the synthesis gas used in the present invention may be less than 5, and preferably less than 2.

When the synthesis gas is used instead of carbon monoxide in the synthesis of N,N' -disubstituted urea, a portion of the mononitro compound reacts with hydrogen to produce a corresponding amine as shown in the following reaction scheme(3):

$$ArNO_2 + 2CO + H_2 \rightarrow ArNH_2 + 2CO_2 \qquad (3)$$

Therefore, consumption of the aromatic mononitro compound is reduced by the reaction(3) and the process economy is improved by using the cheap synthesis gas.

The palladium compounds useful as a main catalyst in this invention are composed of a divalent palladium ion. They can be represented by the following formula(II):

$PdX_2L_2$ (II)

wherein X indicates a halogen, $NO_3$, $OCOCH_3$ or $OCOCF_3$ moiety; and L indicates $PR_3$(wherein R is methyl, ethyl, propyl, butyl, or phenyl), $C_6H_5NH_2$, $CH_3CN$, p-$ClC_6H_4NH_2$, or p-$CH_3C_6H_4NH_2$ as a ligand. The amount of the main catalyst preferably ranges from 1/3000 to 1/10 mole per mole of the aromatic mononitro compound. When the main catalyst of the type $PdX_2$ is used, it is necessary to add one of the aforementioned ligands to the reaction mixture. The amount of the ligand is preferably more than 2 moles per mole of the main catalyst.

The halogen-containing compound used as the co-catalyst may include: ammonium salts of the formula $[R'_4N]^+ X'^-$ and phosphonium salts of the formula $[R'_4P]^+ X'^-$, wherein R' represents a hydrogen, paraffinic, aromatic, or paraffinic aromatic group; and X' represents a halogen atom. Specific examples of such compounds include ammonium salts such as tetramethyl ammonium chloride, tetraethyl ammonium chloride, tetrapropyl ammonium chloride, tetrabutyl ammonium chloride, tetramethyl ammonium bromide, tetraethyl ammonium bromide, tetrapropyl ammonium bromide, tetrabutyl ammonium bromide, tetramethyl ammonium iodide, tetraethyl ammonium iodide, tetrapropyl ammonium iodide, tetrabutyl ammonium iodide, trimethyl benzyl ammonium chloride, etc.; and phosphonium salts such as tetramethyl phosphonium chloride, tetraethyl phosphonium chloride, tetrapropyl phosphonium chloride, tetrabutyl phosphonium chloride, tetramethyl phosphonium chloride, tetraethyl phosphonium bromide, tetrapropyl phosphonium bromide, tetrabutyl phosphonium bromide, tetramethyl phosphonium iodide, tetraethyl phosphonium iodide, tetrapropyl phosphonium iodide, tetrabutyl phosphonium iodide, etc.

The amount of the co-catalyst may range from 1 to 20 moles per mole of the main catalyst, i.e., the palladium compound. When the amount of the co-catalyst is less than 1 mole per mole of the main catalyst, the rate of reaction becomes negligible. On the other hand, if it is more than 20 moles per mole of the main catalyst, it is not economical.

It is preferable to control the amount of the main catalyst and the ligand so as to dissolve them completely in due consideration of the solubility of each component under the reaction conditions such as reaction temperature, pressure, amount of the aromatic primary amine and of the solvent.

The process of the invention may be carried out in the absence of a solvent; however, the use of a solvent is not preculded. Suitable solvents are preferably non-polar solvents such as benzene, toluene and xylene which hardly dissolve N,N' -disubstituted urea.

The reaction temperature is generally held in the range of between 50 and 200 °C, preferably between 80 and 140 °C. When the reaction temperature is lower than the above range, a large amount of mononitro compound remains unreacted. Whereas, if the reaction temperature is higher than the above range, the catalyst tends to be deactivated or decomposed.

Even though it is possible to carry out the reaction at any pressure greater than 101 kPa (1 atm.), the reaction pressure is generally held in the range of between 505 and 10100 kPa (5 and 100 atm.), and preferably between 505 and 4040 kPa (5 and 40 atm.) When the reaction pressure is less than 505 kPa (5 atm.), the reaction rate becomes too slow. When the reaction pressure is more than 10100 kPa (100 atm.), it requires much expensive high pressure equipment.

The reaction time depends on the nature and amount of reactants, the reaction pressure, temperature, and the type and amount of the catalyst used. However, it generally lies in the range of 10 minutes to 10 hours.

After completion of the reaction, the final product, N,N' -disubstituted urea, is recovered as a solid from the reaction mixture by filtration or centrifugation followed by washing. Since the main catalyst, co-catalyst, unreacted materials and solvent are present in the remaining liquid phase, the main catalyst and co-catalyst are almost completely recovered from the mixture by the filtration of product solids. The urea derivatives thus obtained are subjected to a conventional purification procedure including washing and separation under a reduced or elevated pressure, thereby obtaining the product with a high degree of purity. At the same time, the catalyst mixed with the solid product is recovered almost completely. The same aromatic primary amine as the reactant is preferably used for washing, since it can be used for the next reaction without any further treatment.

As shown in Fig. 1, when the present invention is applied to a continous mode, raw materials are charged into the reactor 1. After the reaction is completed, the gases which are mainly carbon monoxide, hydrogen, carbon dioxide, and water vapor are separated from the reaction mixture by a gas separator 2. In the first centrifugal separator 3, the resultant slurry is separated into solid and liquid materials. The solid

material is conveyed to the slurry drum 4 where the solvent for catalyst recovery is charged. The mixture is then thoroughly stirred and separated in the second centrifugal separator 5. The desired N,N' -disubstituted urea is obtained as a solid after drying in the drier 6. The unreacted materials, solvent, main catalyst and co-catalyst from the first and the second centrifugal separators 3 and 5 are collected in the receiving drum 7. The liquid in the receiving drum 7 is recharged into the reactor 1.

Thus the characteristics of the present invention are to use an aromatic primary amine in an amount sufficient to dissolve the catalysts to maintain a reaction pressure which is optimal for the reaction and the catalyst activity, and preferably to use the same aromatic primary amine both as the reactant and as the solvent for catalyst recovery, so as to improve the yield and purity of the desired product remarkably and reuse the catalyst essentially without any loss.

The present invention will now be described in more detail in connection with the following examples which should be considered as being exemplary and not limiting the present invention.

In all of these examples, the reaction was carried out in an electromagnetically stirred autoclave made of Hastelloy C having a capactiy of 300mℓ. The reactants are heated by a heater attached externally to the reactor. After the reaction was completed, the reaction mixture was cooled to a room temperature by a cooling coil installed within the reactor. The reaction products were analyzed by gas chromatography and high performance liquid chromatography (HPLC). In carrying out the gas chromatographic analysis, t-butyl benzene was used as an internal standard.

The yield of N,N' -disubstituted urea was calculated by the following equation:

$$\text{YIELD(\%)} = \frac{2 \times (\text{moles of N,N}' \text{ -disubstituted urea product})}{\text{reacted moles of aromatic mononitro compound and aromatic primary amine}}$$

Example 1

Into a 300mℓ autocalve were charged 6.15g(0.05mole) of nitrobenzene, 27.9g(0.3mole) of aniline, 0.15g of palladium acetate, 1g of triphenyl phosphine, 2g of tetraethyl ammonium chloride, t-butyl benzene(as an internal standard for gas chromatographic analysis), and 60g of xylene. The autoclave was initially purged three times with pressurized synthesis gas at 10100 kPa (100 atm.), whose molar ratio of hydrogen to carbon monoxide was 0.5; and then the synthesis gas pressure of 60 atm. was established at the room temperature. The reaction mixture was heated with stirring and held at 120 °C for 2.5 hours. During the reaction, liquid samples were taken through a sampling valve and analysed by gas chromatography. After completion of the reaction, the reaction mixture was cooled to the room temperature and the gas was discharged from the autoclave. After the filtration of the reaction mixture under a reduced pressure, the precipitates were washed with 18.6g(0.2mole) of aniline, and 50g of xylene, and then dried. As a result, 18. 5g of white precipitate was obtained. Analysis of the remaining solution revealed that the conversion of nitrobenzene was 99.1% and the yield of N,N' -diphenyl urea(DPU) was 97.1%.

Example 2

The procedure of Example 1 was repeated except that the reaction was carried out at 4040 kPa (40 atm.) with the synthesis gas having the hydrogen to carbon monoxide molar ratio of 1. Analysis of the product revealed that the conversion of nitrobenzene was 98.8% and the yield of DPU was 97.0%.

Example 3

The procedure of Example 1 was repeated except that the reaction was carried out at 6060 kPa (60 atm.) with the synthesis gas having the hydrogen to carbon monoxide molar ratio of 2. Analysis of the product revealed that the conversion of nitrobenzene was 98.3% and the yield of DPU was 96.8%.

Example 4

Reaction A:

The procedure of Example 1 was repeated except that the reaction was carried out at 6060 kPa (40 atm.) with carbon monoxide instead of synthesis gas for 1.5 hours. After the filtration of the reaction mixture under a reduced pressure(its filtrate shall be called Supernatant A), the precipitates were washed with 18. 6g(0.2mole) of aniline and then filtered(its filtrate shall be called Supernatant B), and 50g of xylene, respectively and then dried. As a result, 18.4g of white precipitate was obtained. Analysis of the remaining solution revealed that the conversion of nitrobenzene was 100% and the yield of DPU was 97.3%. Analysis by using inductively coupled plasma(ICP) indicated that the palladium component of the catalyst was not present in the product solids. In other words, the entire amount of the catalyst remained in the filtrates.

Reaction B:

Into a 300mℓ autoclave were charged Supernatants A and B obtained from Reaction A and 6.15g-(0.05mole) of nitrobenzene. Then the procedure of Reaction A was repeated. After the filtration of the reaction mixture(its filtrate shall be called Supernatant C), the precipitate was washed with 18.6g of aniline-(0.2mole), filtered again (its filtrate shall be called Supernatant D), washed again with 50g of xylene, and then dried. As a result, 18.5g of white precipitate was obtained. Analysis of the remaining solution revealed that the conversion of nitrobenzene was 100% and the yield of DPU was 98.0%, which indicates no decrease ocurred in the activity of the catalyst.

Reaction C:

Into a 300mℓ autoclave were charged Supernatants C and D obtained from Reaction B and 6.15g-(0.05mole) of nitrobenzene. Then the procedure of Reaction A was repeated. After the filtration of the reaction mixture(its filtrate shall be called Supernatant E), the precipitate was washed with 18.6g of aniline-(0.2mole), filtered again(its filtrate shall be called Supernatant F), washed again with 50g of xylene, and then dried. Analysis of the remaining solution revealed that the conversion of nitrobenzene was 100% and the yield of DPU was 98.0%. Analysis by using ICP indicated that the palladium component of the catalyst was not present in the product solids.

Reaction D:

Into a 300mℓ autoclave were charged Supernatants E and F obtained from Reaction C and 6.15g-(0.05mole) of nitrobenzene. Then the procedure of Reaction A was repeated. As a result, 18.5g of white precipitate was obtained. Analysis of the remaining solution revealed that the conversion of nitrobenzene was 100% and the yield of DPU was 98.0%. ICP analysis showed that no palladium component was present in the product solids.

Example 5

The procedure of Reaction A, Example 4 was repeated except that xylene was replaced with 60g of toluene and that the reaction was carried out at 5353 kPa (53 atm.) and 100°c for 6 hours. Analysis of the remaining solution revealed that the conversion of nitrobenzene was 95.2% and the yield of DPU was 92.4%.

Example 6

Reaction A:

The procedure of Reaction A, Example 4 was repeated except that tetraethyl ammonium chloride-($Et_4NCl$) was replaced with tetrabutyl phosphonium bromide($Bu_4PBr$) and that the reaction was carried out for 4 hours. After the reaction, the product mixture was filtered(its filtrate shall be called Supernatant G), and the precipitate was washed with 18.6g of aniline(0.2mole), filtered again(its filtrate shall be called Supernatant H), washed with 50g of xylene, and then dried. Analysis of the remaining solution revealed that the conversion of nitrobenzene was 97.8% and the yield of DPU was 96.4%. ICP analysis showed that no

palladium component was present in the product solids.

Reaction B:

Into a 300mℓ autoclave were charged Supernatants G and H obtained from Reaction A hereof and 6.15g(0.05mole) of nitrobenzene. Then the procedure of Reaction A, Example 6 was repeated. The conversion of nitrobenzene was 98.4% and the yield of DPU was 97.1%.

Example 7

Reaction A:

The procedure of Reaction A, Example 4 was repeated except that palladium acetate($Pd(CH_3COO)_2$) was replaced with palladium chloride($PdCl_2$) and that the reaction was carried out for 6 hours. After the reaction, the product mixture was filtered(its filtrate shall be called Supernatant I), and the precipitate was washed with 18.6g of aniline(0.2mole), filtered again(its filtrate shall be called Supernatant J), washed again with 50g of xylene, and then dried. Analysis of the remaining solution revealed that the conversion of nitrobenzene was 88.9% and the yield of DPU was 86.1%. ICP analysis showed that no palladium component was present in the product solids.

Reaction B:

Into a 300mℓ autoclave were charged Supernatants I and J obtained from Reaction A, Example 7 and 6.15g(0.05mole) of nitrobenzene. Then the procedure of Reaction A hereof was repeated. The conversion of nitrobenzene was 89.0% and the yield of DPU was 84.0%.

Example 8

Reaction A:

The procedure of Reaction A, Example 4 was repeated except that palladium acetate($Pd(CH_3COO)_2$) was replaced with palladium trifluoroacetate($Pd(CF_3COO)_2$) and that the reaction was carried out at 62 atm. and 100°C for 7 hours. After the reaction, the reaction mixture was filtered(its filtrate shall be called Supernatant K), and the precipitate was washed with 18.6g of aniline(0.2mole), filtered again(its filtrate shall be called Supernatant L), washed with 50g of xylene, and then dried. Analysis of the remaining solution revealed that the conversion of nitrobenzene was 96.2% and the yield of DPU was 93.4%. ICP analysis showed that no palladium component was present in the product solids.

Reaction B:

Into a 300mℓ autoclave were charged Supernatants K and L obtained from Reaction A hereof and 6.15g(0.05mole) of nitrobenzene. Then the procedure of Reaction A, Example 8 was repeated. The conversion of nitrobenzene was 97.0% and the yield of DPU was 94.0%.

COMPARATIVE EXAMPLE 1

The procedure of Reaction A, Example 4 was repeated except that aniline was omitted. It was observed that no reaction did not occur.

COMPARATIVE EXAMPLE 2

The procedure of Reaction A, Example 4 was repeated except that nitrobenzene was omitted. The reaction did not occur.

COMPARATIVE EXAMPLE 3

The procedure of Reaction A, Example 4 was repeated except that tetraethyl ammonium chloride-($Et_4NCl$) was omitted. The conversion of nitrobenzene was 11.4% and the yield of DPU was 11.5%.

COMPARATIVE EXAMPLE 4

The procedure of Reaction A, Example 4 was repeated except that palladium acetate($Pd(CH_3COO)_2$) was replaced with 0.15g of palladium metal and the reaction was carried out at 50 atm. and 100 °C for 4.5 hours. The reaction did not occur.

COMPARATIVE EXAMPLE 5

The procedure of Reaction A, Example 4 was repeated except that tetraethyl ammonium chloride-($Et_4NCl$) was replaced with 2g of potassium chloride(KCl). The conversion of nitrobenzene was 55.5% and the yield of DPU was 55.1%.

COMPARATIVE EXAMPLE 6

The procedure of Reaction A, Example 4 was repeated except that tetraethyl ammonium chloride-($Et_4NCl$) was replaced with 2g of cupric chloride($CuCl_2$) and the reaction was carried out at 55 atm. for 5 hours. The reaction did not occur.

COMPARATIVE EXAMPLE 7

The procedure of Reaction A, Example 1 was repeated except that xylene was replaced with 70mℓ of acetone. The conversion of nitrobenzene was 25.4% and the yield of DPU was 25.1%.

COMPARATIVE EXAMPLE 8

Reaction A:

Into a 300mℓ autoclave were charged 6.15g(0.05mole) of nitrobenzne, 9.3g(0.1mole) of aniline, 0.5g of palladium acetate, 1g of triphenyl phosphine, 2g of tetraethyl ammonium chloride, 5g of tri-n-butyl amine, t-butyl benzene(as an internal standard for gas chromatographic analysis), and 60g of xylene. The procedure of Reaction A, Example 4 was repeated except that the reaction was carried out at 100 °C for 6 hours. After the reaction, the reaction mixture was filtered under a reduced pressure(its filtrate shall be called Supernatant M), washed with 50g of xylene, and then dried. Analysis of the remaining solution revealed that the conversion of nitrobenzene was 92.0% and the yield of DPU was 90.0%.

Reaction B:

Into a 300mℓ autoclave were charged Supernatant M obtained from Reaction A, 6.15g(0.05mole) of nitrobenzene and 9.3g(0.1mole) of aniline. Then the procedure of Reaction A hereof was repeated. The conversion of nitrobenzene was 28.0% and the yield of DPU was 26.0%.

While the present invention has been shown and described with reference to particular embodiments, it will be apparent to those skilled in the art that many changes and modifications may be made without departing from the spirit and scope of the invention as defined in the claims that follow.

## Claims

**1.** A process for preparing N,N' -disubstituted urea of the formula(I)

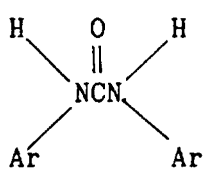

(I)

wherein Ar represents an aromatic radical optionally substituted with a halogen, alkyl, or alkoxy group, which comprises reacting an aromatic mononitro compound, an aromatic primary amine and synthesis gas in the presence of a catalyst consisting essentially of a divalent palladium compound as a main

catalyst component and an ammonium or phosphonium salt containing a halogen atom as a co-catalyst component with or without a solvent.

2. The process of claim 1, wherein the reaction is conducted at a temperature ranging from 50 to 200 °C.

3. The process of claim 1, wherein the reaction is conducted at a pressure ranging from 505 to 10100 kPa (5 to 100 atm.)

4. The process of claim 1, wherein the aromatic primary amine is added to the reaction system in an amount of more than 2 moles per mole of the aromatic mononitro compound.

5. The process of claim 1, wherein the synthesis gas has a hydrogen to carbon monoxide molar ratio of less than 5.

6. The process of claim 1, wherein the palladium compound is represented by $PdX_2L_2$ wherein X indicates a halogen atom, $NO_3$, $OCOCH_3$, $OCOCF_3$; and L indicates $PR_3$ wherein R is methyl, ethyl, propyl, butyl or phenyl, $C_6H_5NH_2$, $CH_3CN$, p-$ClC_6H_4NH_2$ and p-$CH_3C_6N_4NH_2$.

7. The process of claim 1, wherein the co-catalyst is added to the reaction system in an amount ranging from 1 to 20 moles per mole of the palladium compound.

8. The process of claim 1, wherein said ammonium salt is selected from the group consisting of tetramethyl ammonium chloride, tetraethyl ammonium chloride, tetrapropyl ammonium chloride, tetrabutyl ammonium chloride, tetramethyl ammonium bromide, tetraethyl ammonium bromide, tetrapropyl ammonium bromide, tetrabutyl ammonium bromide, tetramethyl ammonium iodide, tetraethyl ammonium iodide, tetrapropyl ammonium iodide and tetrabutyl ammonium iodide.

9. The process of claim 1, wherein said phosphonium salt is selected from the group consisting of tetramethyl phosphonium chloride, tetraethyl phosphonium chloride, tetrapropyl phosphonium chloride, tetrabutyl phosphonium chloride, tetramethyl phosphonium bromide, tetraethyl phosphonium bromide, tetrapropyl phosphonium iodide and tetrabutyl phosphonium iodide.

10. The process of claim 1, wherein the reaction is carried out in the presence of a non-polar solvent.

11. The process of claim 10, wherein said non-polar solvent is selected from the group consisting of benzene, toluene and xylene.

12. The process of claim 1 which further comprises: filtering the reaction mixture to obtain the compound of the formula (I) as a filter cake; and, thereafter, washing said filter cake with said aromatic primary amine in order to completely recover said catalyst.

**Patentansprüche**

1. Verfahren zur Synthese von N,N'-disubstituiertem Harnstoff der Formel (I),

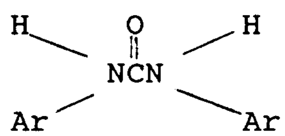

(I)

wobei Ar ein aromatisches Radikal darstellt, wahlweise substituiert mit einem Halogen, einem Alkyl oder einer Alylkoxyl-Gruppe, mit Reaktion einer aromatischen Mononitro-Komponente, eines aromatischen Primäramins und Synthesegas in der Anwesenheit eines Katalysators, welcher im wesentlichen aus einer divalenten Palladium-Komponente als Hauptkatalysatorkomponente und einem Ammonium- oder Phosphoniumsalz besteht, das ein Halogenatom als Ko-Katalysatorkomponente mit oder ohne Lösungsmittel enthält.

2. Verfahren nach Anspruch 1, in welchem die Reaktion bei einer Temperatur in dem Bereich von 50 bis 200 ° C durchgeführt wird.

3. Verfahren nach Anspruch 1, in welchem die Reaktion bei einem Druck zwischen 505 und 10100 kPa (5 und 100 atm) durchgeführt wird.

4. Verfahren nach Anspruch 1, in welchem das aromatische Primäramin dem Reaktionssystem mit einem Anteil von 2 mol pro mol aromatischer Mononitro-Komponente zugesetzt wird.

5. Verfahren nach Anspruch 1, in welchem das Synthesegas ein molares Verhältnis von Wasserstoff zu Kohlenmonoxid von weniger als fünf aufweist.

6. Verfahren nach Anspruch 1, in welchem die Palladium-Komponente durch $PdX_2L_2$ dargestellt wird, wobei X ein Halogenatom, $NO_3$, $OCOCH_3$, $OCOCF_3$ anzeigt; und L $PR_3$ angibt, mit R als Methyl, Äthyl, Propyl, Butyl oder Phenyl, $C_6H_5NH_2$, $CH_3CN$, p-$ClC_6H_4NH_2$ und p-$CH_3C_6N_4NH_2$.

7. Verfahren nach Anspruch 1, in welchem der Ko-Katalysator dem Reaktionssystem mit einem Anteil zwischen 1 und 20 mol pro mol Palladium-Komponente zugesetzt wird.

8. Verfahren nach Anspruch 1, in welchem das Ammoniumsalz aus einer Gruppe ausgewählt wird, bestehend aus Tetramethylammoniumchlorid, Teträthylammoniumchlorid, Tetrapropylammoniumchlorid, Tetrabutylammoniumchlorid, Tetramethylammoniumbromid, Tetraäthylammoniumbromid, Tetrapropylammoniumbromid, Tetrabutylammoniumbromid, Tetramethylammoniumiodid, Tetraäthylammoniumiodid, Tetrapropylammoniumiodid, Tetrabutylammoniumiodid.

9. Verfahren nach Anspruch 1, in welchem das Phosphoniumsalz aus einer Gruppe ausgewählt wird, bestehend aus Tetramethylphosphoniumchlorid, Tetraäthylphosphoniumchlorid, Tetrapropylphosphoniumchlorid, Tetrabutylphosphoniumchlorid, Tetramethylphosphoniumbromid, Tetraäthylphosphoniumbromid, Tetrapropylphosphoniumiodid, Tetrabutylphosphoniumiodid.

10. Verfahren nach Anspruch 1, in welchem die Reaktion in Anwesenheit eines unpolaren Lösungsmittels durchgeführt wird.

11. Verfahren nach Anspruch 10, in welchem das unpolare Lösungsmittel aus einer Gruppe, bestehend aus Benzen, Toluol und Xylen, ausgewählt wird.

12. Verfahren nach Anspruch 1 desweiteren mit:
Filtrieren der Reaktionsmischung, um die Komponente der Formel (I) aus dem Filterkuchen zu erhalten; und danach Waschen des Filterkuchens mit dem aromatischen Primäramin, um den Katalysator vollständig zurückzugewinnen.

## Revendications

1. Procédé de préparation d'urée N,N'-disubstituée de formule (I)

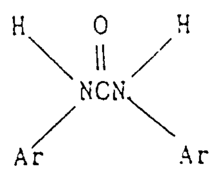

(I)

dans laquelle Ar représente un radical aromatique facultativement substitué par un halogène, un groupe alkyle ou alcoxy, dans lequel on fait réagir un composé mononitro aromatique, une amine primaire aromatique et du gaz de synthèse en présence d'un catalyseur constitué essentiellement d'un composé de palladium bivalent comme composant catalyseur principal et d'un sel d'ammonium ou de phosphonium contenant un atome d'halogène comme composant co-catalyseur avec ou sans solvant.

2. Procédé de la revendication 1, dans lequel la réaction est conduite à une température comprise entre 50 et 200 °C.

3. Procédé de la revendication 1, dans lequel la réaction est conduite à une pression comprise entre 505 et 10100 kPa (5 et 100 atm).

4. Procédé de la revendication 1, dans lequel on ajoute l'amine primaire aromatique au système réactionnel en une quantité supérieure à 2 moles par mole du composé mononitro aromatique.

5. Procédé de la revendication 1, dans lequel le gaz de synthèse a un rapport molaire de l'hydrogène au monoxyde de carbone inférieur à 5.

6. Procédé de la revendication 1, dans lequel le composé de palladium est représenté par $PdX_2L_2$ où X indique un atome d'halogène, $NO_3$, $OCOCH_3$, $OCOCF_3$ ; et L indique $PR_3$ où R est un méthyle, éthyle, propyle, butyle ou phényle, $C_6H_5NH_2$ , $CH_3CN$, p-$ClC_6H_4NH_2$ et p-$CH_3C_6N_4NH_2$.

7. Procédé de la revendication 1, dans lequel le co-catalyseur est ajouté au système réactionnel en une quantité alalnt de 1 à 20 moles par mole du composé de palladium.

8. Procédé de la revendication 1, dans lequel ledit sel d'ammonium est choisi dans le groupe constitué par le chlorure de tétraméthyl-ammonium, le chlorure de tétraéthyl-ammonium, le chlorure de tétrapropyl-ammonium, le chlorure de tétrabutyl-ammonium, le bromure de tétraméthyl-ammonium, le bromure de tétraéthyl-ammonium, le bromure de tétrapropyl-ammonium, le bromure de tétrabutyl-ammonium, l'iodure de tétraméthyl-ammonium, l'iodure de tétraéthyl-ammonium, l'iodure de tétrapropyl-ammonium et l'iodure de tétrabutyl-ammonium.

9. Procédé de la revendication 1, dans lequel ledit sel de phosphonium est choisi dans le groupe constitué par le chlorure de tétraméthyl-phosphonium, le chlorure de tétraéthyl-phosphonium, le chlorure de tétrapropyl-phosphonium, le chlorure de tétrabutyl-phosphonium, le bromure de tétraméthyl-phosphonium, le bromure de tétraéthyl-phosphonium, l'iodure de tétrapropyl-phosphonium et l'iodure de tétrabutyl-phosphonium.

10. Procédé de la revendication 1, dans lequel la réaction est conduite en présence d'un solvant non polaire.

11. Procédé de la revendication 10, dans lequel ledit solvant non polaire est choisi dans le groupe constitué par le benzène, le toluène et le xylène.

12. Procédé de la revendication 1 dans lequel en outre: on filtre le mélange réactionnel pour obtenir le composé de formule (I) sous la forme d'un tourteau de filtration; puis on lave ledit tourteau de filtration avec ladite amine primaire aromatique pour récupérer complètement ledit catalyseur.

Figure 1.

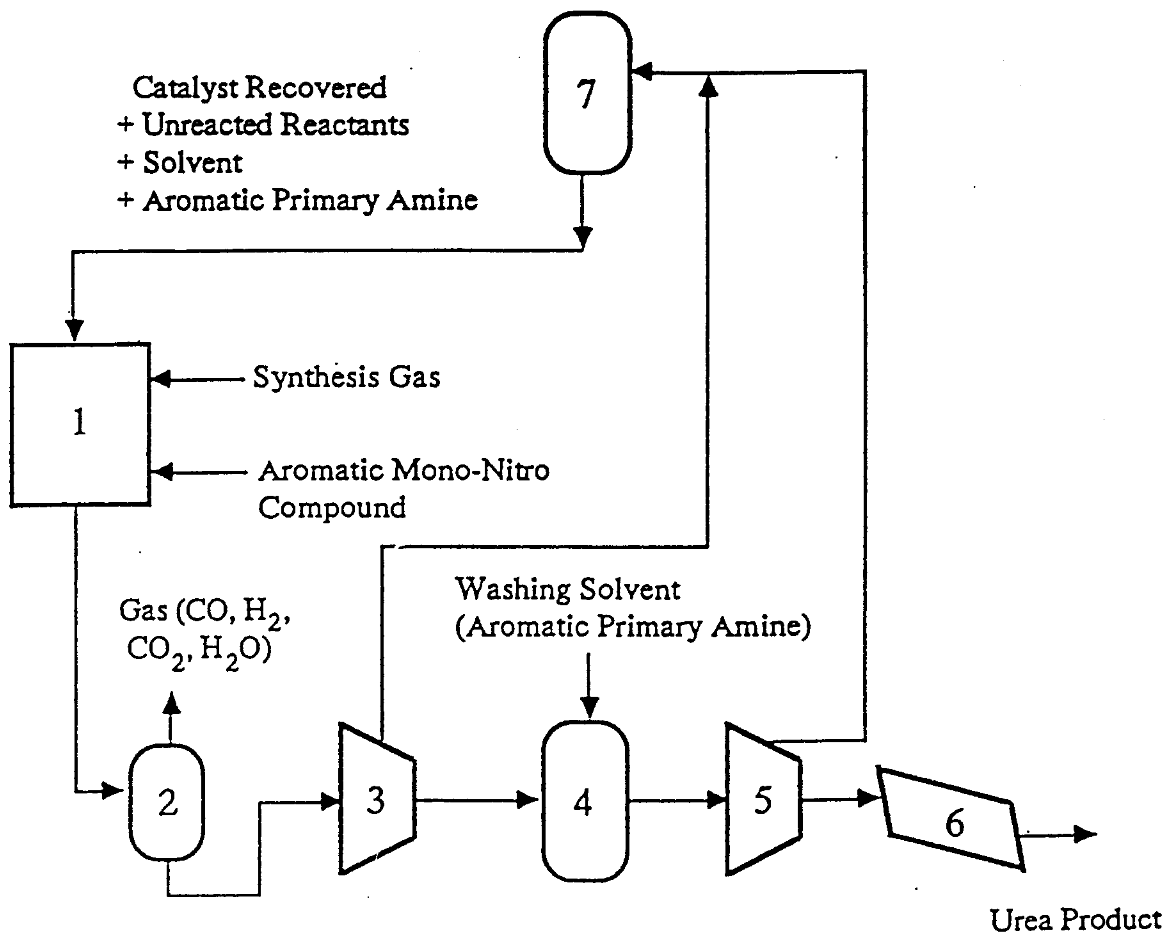
Catalyst Recovered
+ Unreacted Reactants
+ Solvent
+ Aromatic Primary Amine
7
1
Synthesis Gas
Aromatic Mono-Nitro Compound
Gas (CO, $H_2$, $CO_2$, $H_2O$)
Washing Solvent (Aromatic Primary Amine)
2
3
4
5
6
Urea Product